# EUROPEAN PATENT APPLICATION

(11) **EP 0 855 441 A2**
(43) Date of publication of application: **29.07.1998**
(21) Application number: 97122789.7
(22) Date of filing: 23.12.1997
(51) Int. Cl.: C12N 5/08, A01K 67/027

(54) **Isolation and purification of germinal stem cell using magnetic bead functioning as antibody and method for establishing stem cell line**

(30) Priority: 27.12.1996 JP 356643/96
(71) Applicant: DIRECTOR OF NATIONAL INSTITUTE OF ANIMAL INDUSTRY, MINISTRY OF AGRICULTURE, FORESTRY AND FISHERIES, Inashiki-gun, Ibaraki-ken (JP)
(72) Inventor: Imai, Hiroschi, Tsukuba-shi Ibaraki-ken (JP); Tokunaga, Tomoyuki, Ushiku-shi Ibaraki-ken (JP); Takahashi, Seiya, Tsukuba-shi Ibaraki-ken (JP); Yamaguchi, Manabu, Tsukuba-shi Ibaraki-ken (JP); Durcova, Gabriela, 277 21 Libechov (CZ)
(74) Representative: VOSSIUS & PARTNER

(57) **Abstract**

Establishing a method for isolating and purifying undifferentiated germinal stem cell from cell populations as the origin of ES cell or EG cell and a method for generating a stem cell line by growing the cell in vitro.

A method for isolating and purifying germinal stem cell from an embryo or a fetus of a mammalian animal including humans, by utilizing an immunomagnetic bead and an antibody recognizing undifferentiated germinal stem cell, and a method for generating a stem cell line with infinite growth potency and pluripotency, comprising culturing the germinal stem cell in vitro, thereby establishing a cell line thereof.

## Description

### FIELD OF THE INVENTION

The present invention relates to a method for isolating and purifying undifferentiated germinal stem cell from embryos or fetuses in mammals (icluding humans), and a method for preparing a stem cell line having pluripotency but being capable of infinite growth in vitro.

### BACKGROUND OF THE INVENTION

Two types of stem cells derived from the culture of embryonic or fetus cells in vitro, namely ES cell (embryonic stem cell) and EG cell (embryonic germ cell), are known to retain pluripotency but nevertheless infinitely grow as the cells remain undifferentiated.

The ES cell is a culture cell established from the inner cell mass of a murine embryo of age 3.5 days.

Alternatively, the EG cell is a culture cell established from the primordial germ cell of murine fetuses being 8 to 12.5 days pregnant.

These cells belong to cells to be left at their undifferentiated state in the presence of feeder cell and leukemia inhibitory factor (referred to as "LIF" hereinafter); in other words, these cells belong to cells retaining the properties from the stage of inner cell mass to the stage of primordial ectoderm.

A variety of reports have been issued conventionally about the method for establishing an ES cell line. For example, a method has been known, comprising culturing a murine embryo at the blastocyst stage being 3.5 days pregnant by using a fibroblast cell derived from a murine fetus as the feeder cell, thereby establishing cell line (Evans and Kaufman, 1981, Nature, 292, 154 - 156; Martin, 1981, Proc. Natl. Acad. Sci. USA, 78, 7634 - 7638). Additional reports have been published about a method for establishing an EG cell line using a primordial germ cell from a fetus being 8 to 12.5 days pregnant (Matsui, et. al., 1992, Cell, 70, 841 - 847; Labosky, et. al., 1994, Development, 120, 3197 - 3204). Because embryonic cells readily differentiable under conditions in vitro or fetus primordial germ cells containing somatic cells already differentiated are cultured by the culture methods disclosed in these reports, however, these methods are empirical methods at some extent as the method for growing undifferentiated cell. Therefore, the probability of the success in establishing such cell line is low by these methods (Doetschman, et. al., 1985, J. Embryol. Exp. Morphol., 87, 27 - 45; Robertson, 1986, Trends Genet., 1, 9 - 13; McMahon and Bradley, 1990, Cell, 62, 1073 - 1085).

The isolation and subculture of ES cell and EG cell involve extreme difficulty because these cells of themselves are readily differentiable. Additionally, if a great amount of somatic cells other than germ cell is contaminated and grown in the culture system, undifferentiated germ cells are eventually induced into a differentiation state, so that the cells cease to grow finally.

Pesce and De Felici (Pesce and De Felici 1995. Dev. Biol., 170, 722 - 735) have made attempts recently to separate primordial germ cells using magnetic beads and culture the cells in vitro, but they have not yet established EG cell line.

Because these stem cell line have pluripotency, meanwhile, the stem cells injected into a murine embryo at an early stage are incorporated in the normal development process of the embryo, so that they can be differentiated into any cell lineage including germ line, namely endoderm, mesoderm and ectoderm. Under appropriate conditions in vitro, furthermore, the stem cells are induced into differentiation into muscle cells, neuron cells and blood cells.

Concerning chimeras created by using murine ES cell, a great number of reports have been issued, primarily including the report by Bradley, et. al. (Bradley, et. al., 1984, Nature, 309, 255 - 256).

Concerning chimeras generated by using murine EG cell, a number of reports have been published as well (Stewart, et. al., 1994, Dev. Biol., 161, 626 - 628; Labosky, et. al., 1994, Development, 120, 3197 - 3204).

Even a report has been issued (Campbell, et. at., 1996, Nature, 380, 64 - 66) such that an individual can be reconstituted from an embryo-derived cell by nuclear transplantation (Willadsen, 1986, Nature, 320, 63 - 65).

Because these stem cells are possibly grown readily in vitro in an infinite manner, a transgenic animal can be prepared from an ES cell or an EG cell introduced with a gene in the same manner as conventionally carried out for culture cells.

### SUMMARY OF THE INVENTION

It is an object of the present invention to overcome these conventional problems and provide a method for establishing a cell line, by purifying as much as possible undifferentiated germinal stem cell from cell populations as the origin of ES cell or EG cell and then growing the cell in vitro.

The present inventors have made various investigations so as to overcome the problems described above. Consequently, the inventors have established a method for isolating and purifying an objective germinal stem cell derived from an embryo or a fetus of mammals and a method for establishing a stem cell line with infinite growth potency and pluripotency, comprising culturing the resulting germinal stem cell in vitro.

The invention according to claim 1 relates to a method for isolating and purifying a germinal stem cell derived from an embryo or a fetus of a mammalian animal (including humans), by utilizing an immunomagnetic bead functioning as antibody (abbreviated simply as "immunomagnetic bead" hereinbelow) and an antibody recognizing undifferentiated germinal stem cell.

The invention according to claim 5 relates to a method for establishing a stem cell line with infinite growth potency and pluripotency, comprising culturing in vitro the resulting germinal stem cell recovered by a method according to claim 1.

Additionally, the invention according to claim 8 relates to an animal generated by a method for preparing a chimeric animal or by nuclear transplantation, comprising introducing by a gene introduction method an objective gene into the stem cell line established by a method according to claim 5, to prepare a stem cell introduced with the gene and preparing the animal.

### BRIEF DESCRIPTION OF THE DRAWINGS

Fig.1 indicates the change of the recovery rate and purity of ES cells (TT2) from a heterogeneous cell populations with mouse embryonic fibroblast cells at various SSEA-1 antibody concentrations;
Fig.2 indicates the change of the cell number of TT2 in culture at various SSEA-1 antibody concentrations during 3 days culture periods;
Fig.3 indicates the change of the recovery rate and purity of the primordial germ cell at various fetus ages;
Fig.4 indicates the change of the cell number of the primordial germ cells in various culture conditions containing growth factors during 5 days culture periods; and
Fig.5 is a photograph of an established EG cells after alkaline phosphatase staining by an optical microscope (magnification; ×200).

### DESCRIPTION OF THE PREFERRED EMBODIMENTS

The present invention will now be described hereinbelow.

The inventive method according to claim 1 is a method for recovering germinal stem cell of mammals (including humans) from a cell mixture solution of the germinal stem cell with somatic cells and other cells at a high purity.

ES cell is derived from an embryonic cells called as inner cell mass at the blastocyst stage of being 3.5 days pregnant; and EG cell is derived from the primordial germ cells of on day 8 to 12.5 in pregnancy. These cells can be established as cell lines by culturing in vitro. The following explanation is about the establishment of EG cell line by means of an immunomagnetic bead.

More specifically, somatic tissues containing primordial germ cells are collected from fetuses in a mouse on day 8 to 12.5 in pregnancy for culture.

The tissue is cultured in an ES culture medium [D-MEM culture medium (GIBCO, BRL, Inc.) is used as the essential culture medium, which is supplemented with 20 % calf fetus serum (GIBCO, BRL, Inc.), 0.5 % non-essential amino acid solution (GIBCO BRL., Inc.), 0.1 mM β-mercaptoethanol solution (Sigma Chem. Inc.) and 1000 units of LIF (manufactured by Amrad)].

Prior to culturing, the cell is preferably dispersed with an enzyme such as disperse and collagenase.

To the cell mixture solution recovered by the culture is added an antibody. Any antibody recognizing the germinal stem cell may be used herein as the antibody, specifically including SSEA-1 (primary antibody specifically recognizing germinal stem cell; Solter and Knowles, 1978, Proc. Natl. Acad. Sci. USA, 75, 5565 - 5569; Fox, et. al., 1981, Dev. Biol., 83, 391 - 398). Besides, for example, TG-1 (Beverley et. al., 1980, Nature, 287, 332 - 333), 4C9 (Yoshinaga, et. al., 1991, Differentiation, 48, 75 - 82) and EMA-1 and EMA-6 (Hahnel and Eddy, 1986, J. Reprod. Immunol., 10, 89 - 100) may satisfactorily be used.

The concentration of an antibody to be added and the timing for the addition vary depending on the type of the antibody to be added; if the SSEA-1 antibody (Kyowa Co. Ltd) is used, 10 to 20 µg of the antibody is added to a cell mixture solution of 300 µl, for culture under agitation at 4°C for about 45 minutes.

By subsequently adding an immunomagnetic bead coated with an antibody (secondary antibody) recognizing the primary antibody into the cell mixture solution, a complex of the germinal stem cell and the immunomagnetic bead is formed.

Any secondary antibody binding to said primary antibody may be used, satisfactorily, including for example a rat anti-mouse IgM antibody (MiniMACS, Milteny Biotech, Inc.) when the SSEA-1 antibody is used as the primary antibody.

Then, the cell mixture solution containing cells not recognized with the complex or the antibodies is added into a cell separation column (MiniMACS, Milteny Biotech., Inc.) mounted on a permanent magnet, to separate the feeder cell with no antibody bound thereon. Then, the magnet is removed to elute and recover the objective fraction of the germinal stem cell.

The fraction of the germinal stem cell, thus recovered, is cultured in vitro. The method for culturing the fraction of the stem cell in accordance with the present invention will now be described for the case of primordial germ cell.

Firstly, the fraction of said stem cell is cultured in an EG culture medium. The EG culture medium is composed of said ES culture medium containing 20 % calf fetal serum (referred to as "FCS" hereinbelow) (Robertson, 1987, Teratocarcinoma and Embryonic Stem Cells - A practical approach, IRS Press, 71 - 112) as the essential culture medium, which is supplemented with an essential growth factor inevitable for the culture of the primordial germ cell in vitro.

The growth factor herein added is for example LIF (1000 units) and basic fibroblast cell growth factor (referred to "bFGF" hereinbelow; 10 ng/ml). The primordial germ cell cannot be grown without any of these growth factors. Additionally, growth factors such as tumor necrosis factor (TNFα), oncostatin M, CNTF, and interleukin-11 may be used.

In addition to these growth factors, forskoline (10 µM, Sigma Chem. Inc. ) is effectively added. Through the addition of forskoline, the growth of the primordial germ cell is more distinctive.

The culture is carried out by using SI⁴-m220 cell as the feeder cell in a carbon dioxide incubator at 37°C for about 6 to 8 days. During the culture, the culture medium should be exchanged to a fresh one every day. The primordial germ cell isolated and purified after the culture forms colonies specific to undifferentiated cells. In such manner, the primordial germ cell can be isolated and purified at a high purity from somatic tissue.

Then, a method for establishing a stem cell line by preparing a primordial germ cell recovered by the aforementioned method is described below.

The cell fraction recovered by the aforementioned method is cultured. By the same method as the method for culturing primordial germ cell, the fraction is cultured in an EG culture medium [produced by supplementing the ES culture medium with 10 ng bFGM (GIBCO BRL, Inc.) and 10 µM forskoline ] for about 6 to 8 days.

After completion of the culture, the developing colonies are recovered. Then, the cells therein are dispersed and cultured in an ES culture medium containing 20 % FCS and LIF. A cell line can be recovered through the culture. The resulting cell line is frozen for storage.

The cell line thus recovered are derived from the primordial germ cells from several fetuses, and therefore, the cell line is composed of clones of a plurality of stem cells. Thus, the cells are subcloned to generate a homogenous cell line.

The subcloning method is described in the following one example. Firstly, the recovered cell line is cultured in an ES culture medium containing mouse embryonic fibroblast cells as the feeder cells. By the culture, the cells are grown to 80 % confluency. The generated cells are added into a culture dish into which murine embryonic fibroblast cell is preliminarily inoculated, and then, independent colonies are recovered 4 to 5 days later. Subsequently, subculture is carried out. The cells grown through the culture are of homogenous cell line, which can be frozen and stored.

Because the germinal stem cell recovered by the method of the present invention has pluripotency, the cells can absolutely be differentiated into any of endoderm, mesoderm and ectoderm. Under appropriate conditions in vitro, the cells can be induced into differentiation into muscle cells, neuron cells and blood cells.

Using the method described above, the isolation and purification of germinal stem cells of mammalian species, such as human and experimental animals including rat, rabbit, mink, cattle, pig, goat and sheep, in addition to mouse, can be carried out, together with the establishment of a stem cell line.

Even if a great number of cells are induced into a differentiation state during the culture of stem cells, undifferentiated stem cells can be purified by the present method to establish an objective cell line.

Because the stem cell line recovered by the present invention has pluripotency, a gene-introduced (transgenic) animal can be generated by culturing the stem cell line in vitro and introducing a useful gene (for example, tumor suppressing gene) into the cell line according to routine methods to prepare the animal from the stem cell after the gene is introduced therein. In this case, for example, a method for introducing gene comprises constructing a gene of a marker gene resistant to pharmaceutical agents (neomycin resistant gene, hygromycin resistant gene, thymidine kinase gene, diphtheria toxin gene, etc.) linked to the objective gene to be introduced and subsequently introducing the constructed gene into stem cell by electroporation or calcium phosphate method. After the introduction of the gene in such manner, only the stem cells introduced with the gene are selected by means of pharmaceutical agents, to generate an individual animal from these stem cells introduced with the gene by the method for preparing chimeric animal or by the nuclear transplantation.

Herein, the process of generating an individual animal by the method for preparing chimera or the nuclear transplantation is now explained. By introducing about one to five cells of the gene-introduced cell into an early embryo (embryo at the eight-cell stage to the blastocyst stage) or by mixing or contacting the stem cells with zona-free embryo in culture, the gene is introduced into the normal early embryo. The embryo is transplanted into the uterus of a synchronized female (being 2.5 to 3.5 days pregnant if the female is mouse), for delivery. The color of the hair of the resulting neonatal determines whether or not the neonatal is derived from the stem cells.

Therefore, such determination is simply done if the stem cells have a different phenotype from that of the early embryo, as to the color of hair. Additionally, the presence or absence of the introduced gene can be determined in a simple fashion, even by PCR and Southern blot technique. An animal of entire somatic tissues derived from the stem cells can be generated by mating such chimeric animal with each other.

The nuclear transplantation is described below. After removing the cell nucleus of unfertilized egg by means of a glass microtube placed in a micromanipulator, the gene-introduced stem cells are injected into a portion just below the zona pellucida. The unfertilized egg is fused with the stem cells by electrical cell fusion or cell fusion via Sendai virus. The embryo introduced with these stem cells is transplanted into the uterus or oviduct of a synchronized female, to recover the neonatal. The resulting animal is a transgenic animal with the genetic characters of the stem cells because the cell nuclei of the animal has been replaced with the nuclei of the stem cells.

Because the basic procedures for preparing an ES cell strain is common to any mammal, the method of the present invention is applicable to animal species other than mouse, once undifferentiated cells are isolated. For the experimental animals and cattle described above, for example, ES cell lines have been established by the same procedures as for mouse, namely by culturing a cell derived from cells at the blastocyst stage in vitro in a culture system composed of a culture medium supplemented with D-MEM, calf fetal serum and LIF and mouse embryonic fibroblast cell as the feeder cell (for rat: Lannaccome, et. al., 1994. Dev. Biol., 163, 288 - 292; for rabbit: Graves and Moreadith, 1993, Mol. Reprod. Dev., 36, 424 - 433; for mink: Sukoyan, et. al., 1992, Mol. Reprod. Dev., 33, 418 - 431; for cow: Stice. et. al., 1996, Biol. Reprod. 54, 100 - 110; for pig: Wheeler, 1994, Reprod. Fetil. Dev., 6, 563 - 568; for sheep: Handyside, et. al., 1987, Roux's Arch. Dev. Biol., 196, 185 - 190).

Morphologically, the established ES cells cannot be discriminated from the corresponding murine cell line. Further, the activity of protein, for example alkaline phosphatase, is commonly observed in these cells, at the intermediate stage of the culture or after the establishment of the cell line.

So as to apply the immunomagnetic bead method for establishing an ES cell line in mammals other than mouse, for example, the following procedures are carried out; proliferating an embryonic cell derived from the blastocyst stage on mouse embryonic fibroblast cells, dispersing the resulting embryonic cell in a trypsin-EDTA solution and the like, treating the dispersion with an immunomagnetic bead specific to the undifferentiated cell in the same manner as in the aforementioned procedures, isolating and purifying the undifferentiated cell through a column and again culturing the recovered undifferentiated cell on mouse embryonic fibroblast cells. In such manner, the cells differentiated from the embryonic cells can be removed from the culture system, to suppress the induction of differentiation of the undifferentiated cell by the differentiated cells.

Alternatively, an ES cell line has successfully been established from the primordial germ cells in cow (Cherny, et. al., 1994, Reprod. Fertil. Dev., 6, 569 - 575; Strelchenko, 1996, Theriogenology, 45, 131 - 140); a culture system using calf fetal serum and LIF and bFGF as growth factors can be used in the same manner as in the procedures of the present invention or existing procedures (Matsui, et. al., 1992, Cell, 70, 841 - 847; Labosky, et. al., 1994, Development, 120, 3197 - 3204). Furthermore, as in the case of the ES culture medium, the primordial germ cells have also the activity of alkaline phosphatase as one marker protein of undifferentiated cell (Cherny, et. al., 1994, Reprod. Fertil. Dev., 6, 569 - 575). Even in this case, therefore, primordial germ cells can be isolated readily by any available antibody specific to undifferentiated cells, which contributes greatly to the facilitation of the isolation of an EG cell line in an animal species, other than mouse.

The present invention will now be described in detail in the following examples.

### Example 1

### Effects of the concentration of SSEA-1 antibody on the isolation and purification of currently existing ES cell (TT2)

TT2 cell already established (Yagi, et. al., 1993, Anal. Biochem., 214, 70 - 76) was used as an ES cell. It was examined as to whether or not the cell isolation method by means of magnetic bead was effective for the purification of the TT2 cell from a cell mixture solution of the TT2 cell and a feeder cell.

For culturing the ES cell, a feeder cell was firstly prepared. Murine fetuses of being 14 days pregnant were disrupted and dispersed in trypsin and EDTA solution. Then, the resulting dispersion was cultured in D-MEM (GIBCO BRL, Inc.) solution containing 10 % calf fetal serum. 10 µg/ml mitomycin C (manufactured by KYOWA, CO.) was added to the cell culture for 2 hours to inhibit cell prolification, and then, the resulting murine fetus fibroblast cell on termination of the growth was used as the feeder cell.

The TT2 cell was cultured on the feeder cell in an ES culture medium (Robertson, 1987, Teratocarcinoma and Embryonic Stem cell - A practical approach, IRL Press, 71 - 112) containing 20 % FCS (GIBCO BRL, Inc.).

1.5 days after the initiation of the culture, the cell was peeled off in 0.05 % trypsin and 0.01 % EDTA solution and then divided in tubes, each containing 1.5 × 10⁶ cells in total. After centrifugation (at 4°C and 900 rpm for 4 minutes), the cell was suspended in 300 µl of the ES culture medium, and the resulting suspension was treated with an anti-SSEA-1 antibody (manufactured by KYOWA, CO. ) at a concentration of 10 µg/300 µl, 20 µg/300 µl or 50 µg/300 µl.

The cell solution was cultured under slow agitation at 4°C for 45 minutes, prior to centrifugation (at 4°C and 900 rpm for 4 minutes). To the resulting cell mass were subsequently added 300 µl of the ES culture medium and 25 µl of a magnetic bead bound with rat anti-mouse IgM antibody (Milteyl Biotec. Inc.). Subsequently, 200 µl of the ES culture medium was added to the resulting mixture. Fifty microliters of the resulting suspension were kept for counting the number of the ES cell, while the remaining cell suspension was added to a cell separation column (Milteyl Biotec. Inc.). A permanent magnet was arranged on the side face of the column, while the separation column was preliminarily washed in the ES culture medium (1 ml) prior to the addition of the cell. The cell suspension was added dropwise into the uppermost top of the column by means of a Pasteur pipette to be left to stand thereafter. Then, the column was washed in 500 µl of the ES culture medium. The washing solution was recovered in a tube, as cells not adsorbed onto the column (somatic cell fraction).

After recovery, the permanent magnet was removed, to recover the cell and ES culture medium left in the column, by means of an injection syringe (ES cell fraction). One hundred microliters of the recovered fraction were kept for counting the number of the ES cell, while the remaining cells were used for determining the viability and growing potency of the cells after separation.

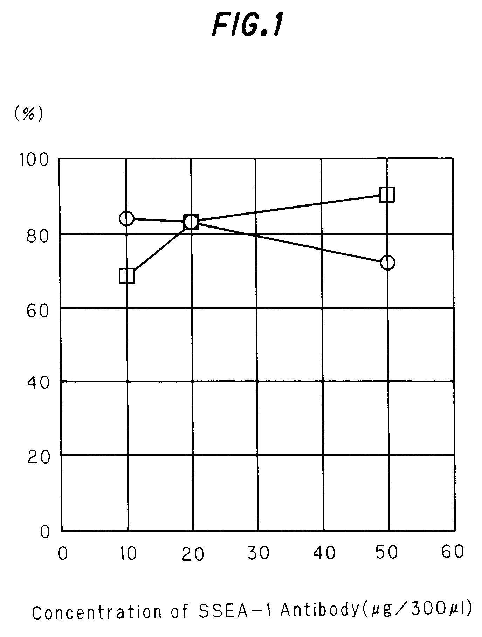

All the cells recovered were cultured in an ES culture medium on murine embryonic fibroblast cell as the feeder cell for one day, to calculate the recovery rate and purity of the TT2 cell, using as the activity of alkaline phosphatase as a marker protein for detecting undifferentiated cell (Chiquoine, 1954, Anat., Rec., 118, 135 - 146; Mintz and Russell, 1957, J. Exp. Zool., 134, 207 - 238). The alkaline phosphatase activity was assayed by Alkaline phosphatase Leucocyte Kit (Sigma Inc.). The results are shown in Fig. 1. In the figure, the symbols □ and ○ represent the recovery rate and purity, respectively.

As apparently shown in the figure, it is indicated that the cell isolation method by means of the magnetic bead can isolate and purify the TT2 cell efficiently.

The recovery rate of the TT2 cell is elevated, depending on the antibody concentration; at a 50 µg/300 µl antibody concentration, a recovery rate as high as 90 % was yielded.

Alternatively, the purity of the TT2 cell in the recovered cell solution was 80 % or more at antibody concentrations of 10 µg/300 µl and 20 µg/300 µl, but the recovery rate was slightly low at the antibody concentration of 50 µ g/300 µl. Thus, a lower antibody concentration was likely to elevate the purity.

Thus, it is indicated that the concentration of the antibody to be used for recognizing the cell was required to be as high as about 20 µg/ml.

### Example 2

Viability of TT2 cell after isolation by the cell isolation method by means of the magnetic bead

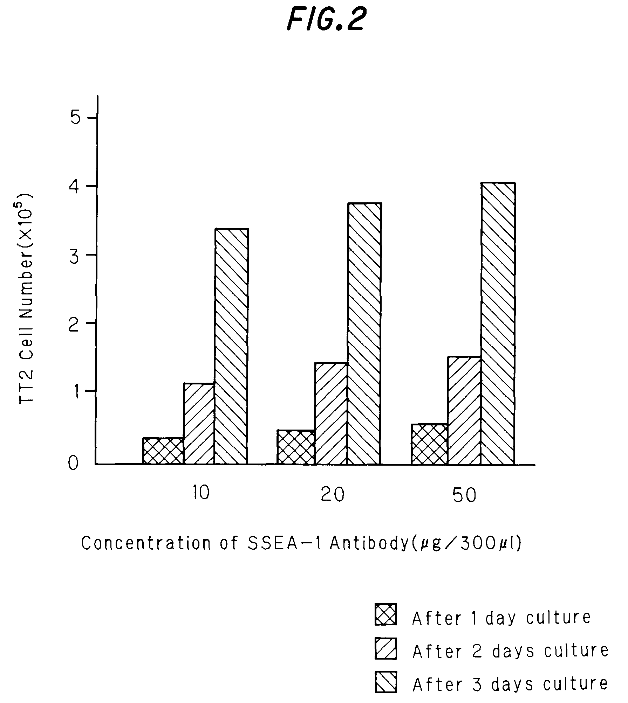

The TT2 cell isolated by the same method as in Example 1 was cultured in an ES culture medium containing murine embryonic fibroblast cell as the feeder cell for one day, two days or three days. Subsequently, the cell number was counted by using as the indicator alkaline phosphatase activity. The results are shown in Fig. 2.

As apparently shown in the figure, it is indicated that the cell growth potency of the cells recovered after labeling at any of the SSEA-1 antibody concentrations was absolutely never modified after the culture in vitro. Additionally, it is indicated that the concentration of the treating antibody, the cell separation column subsequently used, and the cell isolation method with the magnetic bead did not affect the cell growth at all.

### Example 3

### Isolation of primordial germ cells derived from fetus

Using the cell isolation method with the magnetic bead, a fetus-derived primordial germ cells were isolated.

A female ICR mouse (SLC Inc., Japan) was spontaneously mated with a male of the same strain; from the uterus of the mouse being 10.5 to 12.5 days pregnant was drawn out a fetus, of which the abdominal part was incised under a microscope. Being 7.5 days pregnant, primordial germ cell is present on the basal part of allantoic diverticulum; 10.5 days pregnant, the cell is present on the peripheral part of archenteron; 11.5 days pregnant, the cell is present on the gonadal ridge. Accordingly, these tissues were incised out with autopsy scissors and washed twice in a PBS solution (phosphate buffered solution). Herein, 5 to 7 fetuses of age 12.5 days and 5 to 9 fetuses of age 11.5 days and 10.5 days were used.

The resected tissues were washed twice in a PBS solution with no calcium or magnesium contained therein, which were then transferred individually into an Eppendorf tube. The tissues were gradually agitated in 100 µl of PBS containing dispase (1000 units; GIBCO BRL Inc.) and collagenase III (72 units; GIBCO BRL Inc.), to disperse the cells. After subsequent centrifugation of the cell suspensions (at 4°C and 900 rpm for 4 minutes), the resulting cell pellets were individually suspended in 300 µl of the ES culture medium.

By the same procedures as for the purification method of TT2 cell as shown in Example 1, labeling with the SSEA-1 antibody and the magnetic bead and cell purification through the cell separation column were carried out.

The SSEA-1 antibody concentration was 10 µg/300 ml for the fetuses of age 10.5 days or 20 µg/300 ml for the fetuses of age 11.5 days and 12.5 days.

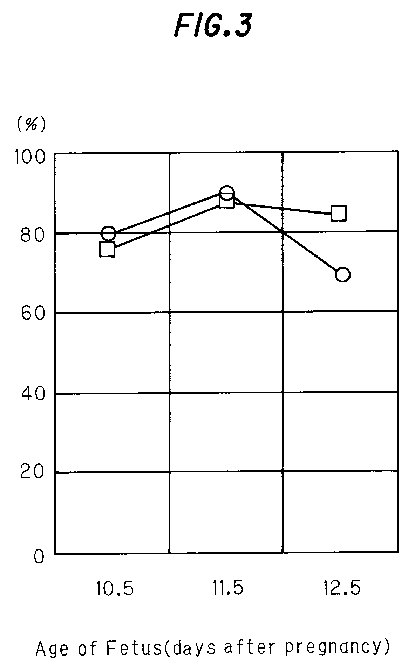

In the same manner as in Example 1, the cell recovery rate and the purity of the primordial germ cell at each stage of cell culture were calculated. The results are shown in Fig. 3. In the figure, the symbols □ and ○ represent the recovery rate and purity, respectively.

As shown in Fig. 3, the recovery rate of the primordial germ cells was as high as around 80 % (78 to 80 %), irrespective of the age in day of any fetus. The purity of the cells from the fetuses of age 12.5 days was slightly low, but a higher purity could be obtained from the fetuses of age 10.5 days and 11.5 days.

This demonstrates that the method of the present invention is practically effective for the separation of primordial germ cells.

### Example 4

### Culture of primordial germ cells with alkaline phosphatase activity in vitro

Among the fractions of primordial germ cells as recovered in Example 3, the fraction from the fetuses (5 to 9 individuals) of age 11.5 days was cultured at 37°C in an atmosphere of 5 % CO₂ and 95 % air in a 4-well culture dish (Nunc Inc.) on Sl⁴-m220 cells capable of producing the membrane-bound stem cell (SCF) (Matsui, et. al., 1991, Nature, 353, 750 - 752) as the feeder cell, for one to 5 days.

The following three types of culture media were used.
1. EG culture medium with no growth factor or forskoline
2. EG culture medium containing LIF alone as growth factor (ES culture medium)
3. EG culture medium (containing 20 % FCS, 1000 units of LIF, 10 ng/ml bFGF and 10 µM forskoline)

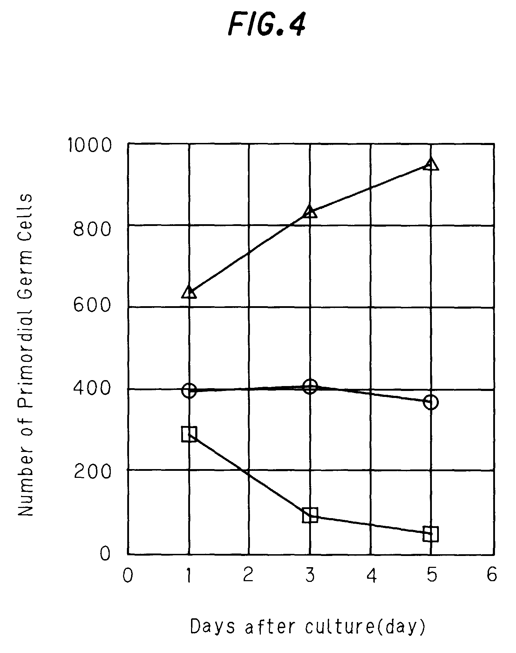

Subsequently, the growth potency of the cell was determined by calculating the alkaline phosphatase activity in the individual culture media for various culture periods. The results are shown in Fig. 4. The symbols □, ○ and ▲ represent the EG culture medium with no growth factor or forskoline contained therein, the EG culture medium containing LIF alone as growth factor (ES culture medium) and the EG culture medium of itself, respectively. Additionally, each culture medium was exchanged to a fresh one, once a day.

The alkaline phosphatase activity of the cell was assayed by washing the cells in a PBS solution, prior to drying in air and fixing, and subsequently adding therein a staining substrate.

As apparently shown in the figure, it is indicated that the number of the separated cells after culture in the culture medium 1 with no growth factor contained therein was decreased as the culture period was longer. The number of the cell cultured in the culture medium 2 containing LIF alone as the growth factor was not specifically changed. In other words, it is indicated that the cell viability was retained, but with no effect on the cell growth.

For a culture period of 5 days, consistent cell growth was observed in the culture medium 3 with addition of all the growth factors and forskoline.

This indicates that the EG culture medium containing LIF, bFGF and forskoline is preferably used for culturing primordial germ cells in vitro.

### Example 5

### Preparation of cell line from primordial germ cell

The colonies recovered after the 5-day culture in the culture medium 3 in the Example 4 were morphologically like as the ES cell. The colonies were recovered by means of a Pasteur pipette and then washed in a PBS solution to disperse the cells therein in a solution containing 0.05 % trypsin and 0.01 % EDTA. The resulting dispersion was continuously cultured in an ES culture medium containing 20 % FCS and LIF (1000 units) and also containing a fibroblast cell prepared from a murine fetus of being 14 days pregnant (Robertson, 1987, Teratocarcinoma and Embryonic Stem Cell - A practical approach, IRL Press, 71 - 112) as the feeder cell.

The culture medium was replaced with a fresh one daily, and depending on the extent of the cell growth, the size of the culture dish was changed from 4-well culture dish (Nunc Inc.) to 12-well culture dish (Falcon Inc.) and 35-mm culture dish (Falcon Inc.). About 2 weeks after the initiation of the culture, the cell growth reached such a stage that the cells might be cultured appropriately in the 35 mm culture dish.

A part of these cells was used for assaying the alkaline phosphatase activity; the remaining cells were suspended in an EG culture medium with addition of 10 % DMSO (Sigma Chem., Inc.), to be then frozen and stored in liquid nitrogen.

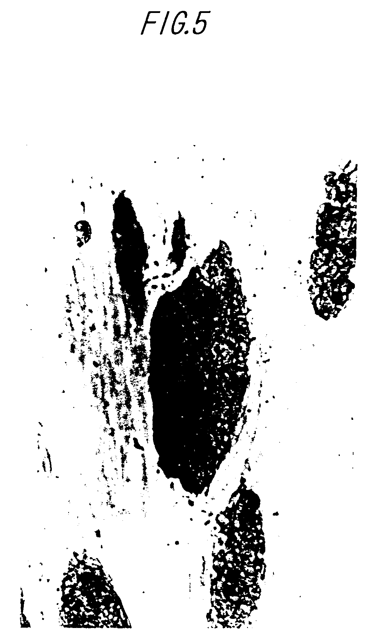

The alkaline phosphatase activity of these cells was assayed. The results are shown in Fig.5.

As shown in the figure, all the colonies exerted high levels of alkaline phosphatase activity. Taking into account that the cells were morphologically identical (to the ES and EG cells), additionally, no difference was observed among the resulting cells and the ES cell and EG cell, previously established.

### Example 6

### Subcloning of EG cell line

The cell line established in Example 5 was derived from the primordial germ cells of the fetuses of 5 to 9 mice. Therefore, a plurality of stem cells were contaminated into the line, which means that a plurality of stem cell lines may possibly be established from the one established cell line.

So as to recover a homogenous cell line, the cell line was then subclomed.

The one cell line recovered in Example 5 was thawed and cultured, in an ES culture medium on murine embryonic fibroblast cell as the feeder cell, to 80 % confluency. The cells grown were washed in a PBS solution and dispersed in 0.1 % trypsin and 0.02 % EDTA, until the cells were separated as a single cell.

These cells were added as a portion of 200 or 1000 cells per 60 mm culture dish (Falcon Inc.) into which murine embryonic fibroblast cells were preliminarily inoculated, to recover independent colonies 4 to 5 days later by means of Pasteur pipette. The colonies were washed in a PBS solution and dispersed in 0.1 % trypsin and 0.02 % EDTA. The dispersion was cultured, together with the feeder cell, in a 24 well culture dish (Falcon Inc.). Following the growth of the cells, the cells were subcultured sequentially in a 12 well culture dish and a 35 mm culture dish, until the cells were appropriately grown in a 60 mm culture dish. The resulting cells were frozen and stored.

After completion of the subcloning, a chromosomal specimen of the cell line was prepared, to examine the karyotype of the cell line.

The specimen was prepared according to the Robertson's method (Robertson, Teratocarcinoma and Embryonic Stem Cell - A practical approach, IRL Press, 71 - 112). More specifically, 10 µl/ml colcemide (GIBCO BRL, Inc.) at the final concentration of 0.1 µg/ml was added to the EG cell grown to 80 % confluency, for culture for 2 hours. The cell was peeled off in 0.1 % trypsin and 0.02 % EDTA, followed by treatment with a low osmotic solution and fixing with methanol and acetic acid. Subsequently, the cell was coated on a slide glass, for staining with a Giemsa staining solution (GIBCO BRL, Inc.), and the chromosome was observed under a microscope.

Among the chromosome specimens prepared for individual experimental groups, at least 50 specimens with a metaphase plate were examined under observation as to the chromosome number and the morphology of the sex chromosome.

The karyotype was examined as to whether the type was normal or not. The results are shown in Table 1.

**Table 1**

| | | Chromosome number | | | Presence or absence of sry gene |
|---|---|---|---|---|---|
| | | 40 | <40 | 40> | |
| Cell line | EG2. 1 | 84% | 12% | 4% | + |
| | EG2. 2 | 88% | 8% | 4% | + |
| | EG2. 3 | 86% | 6% | 8% | + |

Furthermore, in addition to the chromosome specimens, primers for the male chromosome-specific sry gene of the genome DNA purified from the cell were prepared. The sex chromosome of the cell line was determined by PCR (Saiki et. al., 1988, Science, 239, 487 - 491). Primers with nucleotide sequences of the Sequence Nos. 1 and 2 in the Sequence Listing were used then. After PCR the amplified product was composed of 214 bp. The results of PCR are shown in Table 1.

Table 1 indicates that the normal chromosome number in any of these three subcloned cell lines exceeds 80 % (the normal chromosome number is 40 in mice).

This indicates that the cell lines have properties comparative to the normal chromosome number of the murine ES cell and EG cell. As a consequence of PCR, it is indicated that the cell lines have male karyotype.

In accordance with the present invention, stem cells derived from embryos or fetuses of mammals including humans can be recovered at a high purity. Additionally, a technique to efficiently establish a stem cell line from the recovered stem cells in a stable manner is provided.

The present invention provides a fundamental technique for preparing a transgenic animal, which is applicable to mammals other than mouse. Hence, the present invention can make remarkable contributions to the modification of domestic species and the generation of a model animal for genetic disorders and gene therapy in the clinical field.

The entire disclosure of Japanese Patent Application No. 8-356643 filed on December 27, 1996 including specification, claims, drawings and summary are incorporated herein by reference in its entirety.

## Claims

1. A method for isolating and purifying a stem cell from an embryo or a fetus, which comprises utilizing an immunomagnetic bead and a primary antibody which recognizes undifferentiated stem cell

2. The method of claim 1, further comprising culturing an embryo-derived or fetus-derived cell in an ES culture medium containing calf fetal serum, thereafter labelling the cell with a primary antibody, reacting the labelled cell with a magnetic bead bound with a secondary antibody which recognizes the primary antibody, and adding the resulting cell into a cell separation column, thereby and separating and recovering the stem cell from cells not recognized by the primary antibody.

3. The method of claims 1 or 2 wherein the stem cell is either an embryonic stem cell (ES), or an embryonic germ cell (EG).

4. The method of any one of claims 1 to 3 wherein said primary antibody is SSEA-1.

5. The method of any one of claims 1 to 4 wherein said secondary antibody is an anti-mouse IgM antibody.

6. The method of any one of claims 1 to 5 wherein said stem cell is derived from a mammallain, preferrably a mouse, a bovine, or a human source.

7. A method for generating a stem cell line with infinite growth potency and pluripotency, which comprises culturing in vitro a germinal stem cell recovered according to the method of any one of claims 1 to 6, thereby establishing the cell line thereof.

8. The method according to claim 7, wherein the germinal stem cell is cultured in a culture medium comprising fetal serum and a growth factor for stem cells.

9. A method for generating a transgenic animal from a stem cell line containing a target gene, which comprises introducing the target gene into said stem cell line recovered by the method accoriding to claim 8.

10. An animal generated by the method according to claim 9, using the method of chimeric animal preparation or nuclear transplantation.


SEQUENCE LISTING

(1) GENERAL INFORMATION:

(i) APPLICANT:
(A) NAME: Director of National Institute of Animal
Industry, Ministry of Agriculture, Forestry and
Fisheries
(B) STREET: 2, Ikenodai, Kukizaki-cho, Inashiki-gun
(C) CITY: Ibaraki-ken
(E) COUNTRY: Japan
(F) POSTAL CODE (ZIP): none

(ii) TITLE OF INVENTION: ISOLATION AND PURIFICATION OF GERMINAL STEM
CELL USING MAGNETIC BEAD FUNCTIONING AS ANTIBODY AND
METHOD FOR ESTABLISHING STEM CELL LINE

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97 12 2789.7
(vi) PRIOR APPLICATION DATA:
(A) APPLICATION NUMBER: JP 356643/1996
(B) FILING DATE: 27-DEC-1996

(2) INFORMATION FOR SEQ ID NO: 1:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(iii) HYPOTHETICAL: YES

(xi) SEQUENCE DESCRIPTION: SEQ ID NO: 1:

AAGCGACCCA TGAATGCATT TCATGGTGTG TGGT 34

(2) INFORMATION FOR SEQ ID NO: 2:

(i) SEQUENCE CHARACTERISTICS:
(A) LENGTH: 38 base pairs
(B) TYPE: nucleic acid
(C) STRANDEDNESS: single

(D) TOPOLOGY: linear

(ii) MOLECULE TYPE: other nucleic acid
(A) DESCRIPTION: /desc = "synthetic DNA"

(iii) HYPOTHETICAL: YES

(xi) SEQUENCE DESCRIPTION: SEQ ID NO: 2:

GAGGTCGATA CTTATAGTTC GGGTATTTCT CTCTCGTG 38